# EUROPEAN PATENT SPECIFICATION

(11) **EP 3 019 605 B1**
(45) Date of publication and mention of the grant of the patent: **26.06.2019**
(21) Application number: 14823085.7
(22) Date of filing: 08.07.2014
(51) Int. Cl.: B01D 15/38, B01J 20/26, C12N 11/08, B01D 15/08, C12N 11/06, C07K 17/06, C07K 17/08, B01J 20/24, B01J 20/286, B01J 20/32, C07K 1/22, C07K 17/04, C12N 9/10, C12N 11/02, C12N 11/10

(54) **A COMPOSITE CARRIER FOR IMMOBILIZATION OF PROTEINS, POLYPEPTIDES OR OLIGOPEPTIDES, PREPARATION METHODS AND APPLICATION THEREOF**
VERBUNDSTOFFTRÄGER ZUR IMMOBILISIERUNG VON PROTEINEN, POLYPEPTIDEN ODER OLIGOPEPTIDEN, HERSTELLUNGSVERFAHREN UND VERWENDUNG DAVON
SUPPORT COMPOSITE POUR L'IMMOBILISATION DE PROTÉINES, DE POLYPEPTIDES OU D'OLIGOPEPTIDES, PROCÉDÉS DE PRÉPARATION ET APPLICATION CORRESPONDANTS

(30) Priority: 08.07.2013 CN 201310284535
(43) Date of publication of application: 18.05.2016
(73) Proprietor: BioRight Worldwide Company Limited, Road Town, Tortola (VG)
(72) Inventor: CHEUNG, Chung Hong, Hong Kong (CN); WANG, Jun, Hong Kong (CN)
(74) Representative: Carpmaels & Ransford LLP
(86) International application number: PCT/CN2014/081843
(87) International publication number: WO 2015/003622

(56) References cited:
- EP-A1- 1 970 443
- WO-A1-97/41216
- CN-A- 102 068 965
- JP-A- H0 319 687
- US-A- 4 918 016

## Description

### Background of the Invention

The present invention relates to a technical field of immobilization of proteins, polypeptides or oligopeptides. More specifically, the invention relates to preparation and application of a composite carrier used for immobilization of proteins, polypeptides or oligopeptides.

### Background Art

With the advance of biotechnology, the application of enzymes becomes popular. In industry and other fields, enzymes are often used in an immobilized form due to the high cost of free enzymes. Also, the immobilized form is easy to separate and recover from products for repeated usage and is more stable than a free enzyme. Many immobilization methods, such as physical adsorption, affinity linkage, covalent crosslinking, flocculation and encapsulation, are known to obtain the immobilized form of enzymes (Roger A. Sheldon, 2007, Advanced Synthesis & Catalysis, 349: 1289-1307).

Specific activity which is the activity of unit weight of an immobilized enzyme/cell is used to assess the efficiency of the immobilized enzyme/cell. The specific activity is affected by both the immobilization method and specific surface area of the immobilized enzyme/cell. Generally, larger specific surface area results in higher specific activity. Many immobilization methods therefore are developed to increase the specific surface area of the enzyme particle. Nowadays, the common methods to increase the specific surface area include using a porous small-sized carrier. For example, a small particulate carrier having prefabricated capillary pores is used to adsorb or hold enzymes and cells. However, the porosity of the conventional carrier, which makes from hard inorganic or organic materials, is mainly embodied on its surface. The carrier is easily cracked if there are too many internal pores.

Consequently, the scientists in the field are searching for immobilized carriers with large specific surface area and less prone to break.

China patent publication CN1982445A discloses a carrier used for enzyme/cell immobilization and the immobilization method using the carrier. The carrier has large specific surface area, and is physically and chemically stable. However, it is an inert carrier, having no reactive functional group, cannot directly immobilize proteins, polypeptides or oligopeptides. Practically, immobilization of certain proteins or polypeptides (e.g. transglucosidase or protein A) is not effective. In addition, the immobilization cannot immobilize oligopeptides (e.g. glutathione or polymyxin B)
European patent application EP 1970443 discloses a carrier made from an organic foam having open pores for enzymes or cells immobilization and the methods for preparing immobilized enzymes or cells. The process uses flocculation and crosslinking technology to immobilize enzyme protein or cells on the organic foam material.

Affinity chromatography technology, which is a common separation method, utilizes immobilization technique. The principle of affinity chromatography is based on a highly specific reversible interaction between a bioactive substance and its corresponding ligand. The general procedure of the affinity chromatography includes: (1) immobilization of a ligand corresponding to a target bioactive substance onto a carrier column; (2) loading of the target bioactive substance to be purified into the column; (3) de-association of the target bioactive substance from the column by changing the composition of mobile phase such as pH, ionic strength, concentration as well as addition of a competitive ligand to weaken the affinity binding. Affinity chromatography is extensively used as it provides a simple, fast and specific separation. In addition, it offers high resolution. The existing carriers of affinity column are often made from agarose. However, its industrial applications are largely limited because of and the high cost of agarose and a toxic agent, cyanogen bromide (CNBr), which is required for the activation of agarose when coupled with its ligand protein.

### Summary of the Invention

To solve the shortcomings of the existing immobilization techniques, the invention provides a preparation and application of a composite carrier, used for immobilization of proteins, polypeptides or oligopeptides.

Specifically, the invention provides the following items:
A method for preparation of a porous composite carrier for immobilization of a protein, polypeptide or oligopeptide, wherein the method comprises the following steps: a) providing a porous organic foam material wherein the organic foam material is melamine foam; b) precipitating a chitosan onto a surface of walls of one or more pores of the porous organic foam material by reacting with an alkaline; and c) crosslinking the precipitated chitosan with a polyaldehyde compound to form the composite carrier which comprises a crosslinked product having aldehyde groups and immobilized on the surface of the walls of one or more pores of the porous organic foam material, wherein the aldehyde groups are able to react with the protein, polypeptide or oligopeptide.

A porous composite carrier for immobilization of a protein, polypeptide or oligopeptide which is formed by the method above.

A complex, comprising: the composite carrier and a protein, polypeptide or oligopeptide immobilized onto the composite carrier, wherein the protein, polypeptide or oligopeptide is immobilized onto the composite carrier through the reaction between the amino groups of the protein, polypeptide or oligopeptide and the aldehyde groups of the composite carrier.

A method for preparing the complex, wherein the complex is prepared by a reaction between the composite carrier and a solution of a protein, polypeptide or oligopeptide.

Furthermore, a biocatalytic method, a method for killing bacteria or inhibiting bacterial growth, and a method for purifying a glutathione S-transferase, or monoclonal or polyclonal antibody, all using the complex.

In comparison to the existing technology, the invention has following advantages:
1. Firstly, the composite carrier carries reactive aldehyde groups that are able to immobilize stably a protein, polypeptide or oligopeptide through the chemical reaction between the aldehyde groups and amino groups of the protein, polypeptide or oligopeptide. For example, the composite carrier stably immobilizes, through a covalent bond, transglucosidase, polymyxin B, glutathione, as well as protein A. Secondly, the invention uses a porous organic foam material containing open pores that greatly increases the specific surface area and consequently increases the specific activity of the immobilized protein, polypeptide or oligopeptide. Thirdly, the carrier uses inert materials which are cheap and therefore reduces the production cost. Fourthly, the products obtainable in the present invention can be tailor-made to various shapes and sizes without changing the specific surface area, which is especially useful for large scale industrial production.
2. The composite carrier of the present invention can be broadly used for applied enzymology and the field of protein, polypeptide or oligopeptide. Theoretically, the method of the present invention is applicable for immobilization of any protein, polypeptide or oligopeptide which is capable of coupling to an aldehyde group. Moreover, the composite carrier is suitable for large scale industrial production owing to simple preparation and low production cost.
   The composite carrier of the present invention can be made as a reel-shaped cylinder suitable for a packed-bed immobilized column, which can be assembled simpler and more efficient than the conventional particulate-shaped immobilized proteins, polypeptides or oligopeptides. Such advantage is obvious especially in large scale industrial production. The reel-shaped carrier can be directly assembled into a packed-bed column without a tubular axle and a casing. This structure can obviously lower the fixed cost. The packed-bed reaction columns solely consisted of the reel-shaped carriers can be set in series or in parallel and also, the number and length of packed-bed reaction columns can be easily adjusted according to the industrial production scale. In addition, the activity of immobilized protein, polypeptide, or oligopeptide gradually decreases during the reaction, especially those near the entrance of substrates and results in activity difference between the two ends of the packed-bed reaction column. Replacement of low activity packed-bed reaction column with high activity packed-bed reaction column can be done easily so that the life span and productivity of the column can be adjusted more efficiently. This effect cannot be achieved by the conventional particulate-shaped immobilized proteins, polypeptides or oligopeptides.
3. The biocatalytic and affinity chromatographic methods of the present invention are simpler and lower in cost than the conventional technology, and thus are especially suitable for large scale industrial production.

### Brief Description of the Drawing

Fig. 1 shows the results of agar plate after incubation E. *coli* in the presence of immobilized polymyxin B, wherein (A) is the agar plate of sample A; (B) is the agar plate of sample B (control experiment).
Fig. 2 shows the SDS-PAGE of purification of glutathione S-transferase by immobilized glutathione, wherein lane 1 is the glutathione S-transferase before the purification; lane 2 is the glutathione S-transferase after the purification.
Fig. 3 shows the SDS-PAGE of affinity purification of rabbit serum by immobilized protein A column, wherein lane 1 is the rabbit serum before the purification; lane 2 is the rabbit serum after the purification.

### Detailed Description of the Invention

The specific embodiments presented below are for illustration of the invention only and are not intended to be regarded as the limitation of the invention. The term "melamine foam" in the present invention refers to a foam material made from melamine resin. The foam material is soft, has high hydrophilicity, and its density is not greater than 0.02 g/cm³. It also possesses a porous structure of three-dimensional network containing open pores. The material can be prepared in various sizes and different shapes such as granules, straps, sheets, blocks and columns.

The inventors have performed a lot of researches on the immobilization of proteins, polypeptides, or oligopeptides, such as treatment of organic foam materials, and screening of different organic foam materials to select more suitable and better carriers for immobilizing proteins, polypeptides or oligopeptides. The inventors have discovered surprisingly that proteins, polypeptides or oligopeptides can be readily and stably immobilized onto the pore walls of porous organic foam material, if the pore walls have reactive aldehyde groups introduced by chitosan. Based on these findings, the technical solutions of the present invention are developed.

In particular, the present invention provides the following 6 sections:

### [1]. Composite carriers for immobilization of proteins, polypeptides or oligopeptides

The invention provides a composite carrier for immobilization of a protein, polypeptide or oligopeptide. The composite carrier is porous and comprises:
a) a porous organic foam material containing open pores; and
b) a crosslinked product having aldehyde groups and immobilized on the surface of the walls of one or more pores of the porous organic foam material, wherein the aldehyde groups are able to react with the protein, polypeptide or oligopeptide, and the crosslinked product is formed by chitosan via a crosslinking reaction with polyaldehyde compounds.

It is well known in the art that the chemical name of chitosan, a deacetylated product of chitin, is poly-β-(1→4)-2-amino-2-deoxy-D-glucose. Generally, the deacetylated chitin is classified as chitosan if the degree of deacetylation is greater than 55%; or if 1% deacetylated chitin is soluble in 1% acetic acid or 1% hydrochloric acid. In fact, the deacetylated chitin with degree of deacetylation greater than 55% is soluble in the dilute acid. The industrial grade of chitosan has the degree of deacetylation greater than 70%. The degree of deacetylation in the range of 55%-70%, 70%-85%, 85%-95% and 95%-100% is classified as low deacetylated chitosan, middle deacetylated chitosan, high deacetylated chitosan and ultra high deacetylated chitosan, respectively. The chitosan used in the present invention is not limited to a particularly degree of deacetylation. Chitosan of any degree of deacetylation can be used, as long as it can be precipitated onto the pore walls of porous organic foam materials by reducing its solubility with an alkaline or other methods.

"Protein" is a high molecular compound consisting of one or more chains of polypeptide formed by dehydration condensation of amino acids and the polypeptide chains are folded into a specific three-dimensional shape. "Polypeptide" refers to a substance consisting of 20 or more amino acids linked by peptide bonds. A peptide chain which consists of 2-20 amino acids linked by peptide bonds is called an "oligopeptide".

The porous organic foam material is melamine foam. Preferably, the composite carrier for immobilization of a protein, polypeptide or oligopeptide is in a form of granules, straps, blocks, sheets, or columns.

The invention utilizes a porous organic foam material containing open pores as a carrier to increase the specific activity of the immobilized protein, polypeptide or oligopeptide. The carrier also has open pores inside, that is, the pores are not closed and there are at least two inter-connected pores inside the carrier. Application of this carrier enables the immobilized products to possess a structure of three-dimensional network containing open pores, and the reaction solution can flow through the inside of the immobilized products. It not only increases the specific surface area significantly, but also minimizes the variation of the reaction rate between the surface and internal of the conventional granular immobilized products. Moreover, the immobilized products in the present invention differ from the existing products having granular or sheet shape in: they can be prepared in a form of granules, blocks, sheets or other shapes in any sizes as needed and hardly affects the specific surface area.

The carrier in the present invention is made of porous organic foam material with open porous and hydrophilicity properties. The open pores of the carrier facilitate the entry of a solution of proteins, polypeptides or oligopeptides into the internal of the carrier during immobilization, promote the interactions between substrates and proteins, polypeptides or oligopeptides during the reaction, and also facilitate the mass transfer of products after the reaction. The hydrophilic property of the carrier enhances even distribution, proper adhesion and immobilization of the proteins, polypeptides or oligopeptides onto the surface of pore walls of carrier.

The open porous and hydrophilic properties of the porous organic foam material facilitate: (i) the entry of protein solution/enzyme solution/polypeptide solution/oligopeptide solution into the internal of the carrier and their even distribution on the surface of the carrier during immobilization; (ii) the close contact between the substrate and immobilized enzyme during an enzymatic reaction; and (iii) the effect binding between the target compound in the mobile phase and its corresponding ligand on the carrier during chromatographic purification. The composite carrier for immobilization of a protein, polypeptide or oligopeptide in the present invention can be prepared in various sizes and different shapes such as granules, blocks, sheets or other shapes.

### [2]. A method for preparation of the composite carriers for immobilization of proteins, polypeptides or oligopeptides

The present invention provides a method for preparation of the composite carriers mentioned in Section [1], wherein the method comprises the following steps:
a) providing a porous organic foam material containing open pores, wherein the organic foam material is melamine foam;
b) precipitating a chitosan onto a surface of walls of one or more pores of the porous organic foam material by reacting with an alkaline; and
c) crosslinking the precipitated chitosan with a polyaldehyde compound to form the composite carrier which comprises a crosslinked product having aldehyde groups and immobilized on the surface of the walls of one or more pores of the porous organic foam material, wherein the aldehyde groups are able to react with the protein, polypeptide or oligopeptide.

In step b), chitosan reacts with an alkaline to precipitate onto the surface of the walls of one or more pores of the porous organic foam material, and the alkaline used is preferably sodium hydroxide. In step c), upon the crosslinking reaction, reactive aldehyde groups of the composite carrier are obtained, due to the unreacted aldehyde groups of the polyaldehyde, wherein the polyaldehyde is selected from the group consisting of glutaraldehyde and dialdehyde starch.

A specific embodiment of the preparation method of the composite carrier is shown as follows:
a) the porous organic foam material is cut into sheets or granules;
b) an appropriate amount of chitosan solution (0.01%-10% (w/v)) is added into the porous organic foam material, and the foam is pressed to distribute the chitosan solution evenly on the foam;
c) the carrier in step b) is immersed in an alkaline solution (0.01 M-10 M sodium hydroxide) to precipitate chitosan onto the pore walls of the porous organic foam material;
d) after a period of appropriate time, the carrier in step c) is washed by deionized water until it is in neutral pH;
e) the carrier in step d) is immersed in polyaldehyde solution (0.01%-30% (v/v));
f) after a period of appropriate time, the carrier in e) is washed by neutral phosphate buffer (0.001 M-10 M) to remove the unreacted polyaldehyde solution.

### [3]. Complex

The invention also provides a complex consists of the composite carrier described in Section [1], and a protein, polypeptide or oligopeptide immobilized onto the composite carrier.

The protein, polypeptide or oligopeptide is immobilized onto the composite carrier described in Section [1] via a reaction between the amino groups of the protein, polypeptide or oligopeptide and the aldehyde groups on the composite carrier described in Section [1].

The principle of the reaction is: the protein, polypeptide or oligopeptide is immobilized covalently onto the composite carrier described in Section [1] through a schiff base formed by a reaction between the amino groups on the protein, polypeptide or oligopeptide and the aldehyde groups on the crosslinked product of the chitosan and polyaldehyde compound.

Above-described principle shows that, the protein, polypeptide or oligopeptide is immobilized onto the composite carrier, consisted of a porous organic foam material containing open pores, a chitosan and a polyaldehyde compound through chemical reaction. The immobilization process does not change the solubility of the protein, polypeptide or oligopeptide. Different from the present application, China patent publication No. CN1982445A described a method wherein a protein is deposited in the pores of the porous melamine foam by changing the solubility of the protein with a flocculant and a crosslinking agent.

Preferably, the protein, polypeptide or oligopeptide is an enzyme. There is no limitation to the type of the enzyme, any enzyme capable of reacting with aldehyde groups is included in the present invention. For example, one preferred enzyme in the present invention is transglucosidase, but the invention is not limited to it.

The complex is applied to kill bacteria or inhibit bacterial growth. There is no limitation to the type of the protein, polypeptide or oligopeptide, any protein, polypeptide or oligopeptide capable of reacting with aldehyde groups is included in the present invention. For example, one preferred protein, polypeptide or oligopeptide in the present invention is polymyxin B, but the invention is not limited to it.

The complex is applied for affinity purification, wherein the protein, polypeptide or oligopeptide of the complex possesses a specific and reversible binding property with the corresponding bioactive substance. There is no particular limitation to the type of the protein, polypeptide or oligopeptide, any protein, polypeptide or oligopeptide capable of reacting with aldehyde groups is included in the present invention. For example, one preferred protein, polypeptide or oligopeptide in the present invention is glutathione or protein A.

Preferably, the shape of immobilized protein, polypeptide or oligopeptide is sheet or column. More preferably, the sheet-shaped immobilized protein, polypeptide or oligopeptide is cut into disc-shaped and packed as a chromatographic column/immobilized enzyme column, or is rolled into a cylindrical reel structure to form a chromatographic column/immobilized enzyme column.

The granular form of the immobilized protein, polypeptide or oligopeptide prepared in the present invention can be used in a stirred tank reactor or packed-bed reactor. The sheet-shaped products can be rolled into a cylindrical reel structure and directly be used as the reaction column. Such reel-structured products can be used as modules, and packed and disposed inside the bioreactor. Each of the reel-structured products can be used alone or in combination with each other to form reaction columns with adjustable diameter or length for industrial production.

The tension generated from the rolling operation and the continuous pressure applied to the porous material against the inner core during the rolling of the sheet-shaped products of the present invention results in modest compression of the porous material. This rolling process is therefore a process of formation of the reel-structured product and also a process of compression of the porous material. The degree of the tightness of the reel structure can be adjusted by adjusting the pressure applied to the porous material. The diameter of the reel structure can be controlled by changing the numbers of the rolling and the thickness of the carrier material. The height of the reel cylinder can be controlled by changing the width of the carrier or by cutting the reel structure into the required height directly.

The columnar surface of the described cylindrical reel column in the present application can be fixed and sealed by a suitable packing material to prevent loosening of the windings. When the product is filled into a packed-bed column with a tubular axle and a casing, the packing material should be made of a hydrophilic and swelling material such as dried PVA foam and pulp foam, if the immobilized protein, polypeptide or oligopeptide itself does not have said properties in order to reduce the gap that may exist between the column surface and the inner wall of the column holder after filling the reaction tank with water. Such gap can also be tamped with a granular material. The cylindrical reel column should be wrapped with a waterproof and thermal insulation material such as polyurethane foam to reduce energy consumption when it is used directly as a reactor column alone or linked together with other columns without column holder. On the other hand, if the cylindrical reel column shrinks and its volume reduces during the reaction, the packing material, should possess a property of continuous shrinkage, such as rubber or material containing rubber to avoid the development of the gap during the column operation.

When cylindrical reel column is used alone or linked together with other columns, both ends of the column should have a device that connects the column and tubes to allow the efficient inflow and outflow of the reaction solution and have a function of filtration. When used in tandem, the joints of the module blocks of the cylindrical reel columns should be sealed with rubber band to prevent liquid leakage.

### [4]. Preparation of the complex

The invention provides a method for preparing the complex described in Section [3], wherein the complex is prepared by a reaction between the composite carrier described in Section [1] and a solution of a protein, polypeptide or oligopeptide.

The principle of the reaction is: the protein, polypeptide or oligopeptide is immobilized covalently onto the composite carrier through the schiff bases formed between the amino groups of the protein, polypeptide or oligopeptide and the aldehyde groups of the crosslinked product, which is obtained by the crosslinking reaction of chitosan with polyaldehyde.

During the immobilization of the protein, polypeptide or oligopeptide, the carrier can be pressed by hand or a device or machine. Generally, the concentration of the protein, polypeptide or oligopeptide used is 0.3%-30% (w/v), and the concentration of the crosslinking agent is 0.01%-30% (v/v). The crosslinking agent is usually a polyaldehyde compound (e.g. glutaraldehyde, dialdehyde starch, glucan dialdehyde, etc.).

### [5]. Application of the complex

The present invention also provides the application of the complex, described in Section [3], wherein the application includes biocatalyst and affinity chromatography.

Preferably, the protein, polypeptide or oligopeptide is an enzyme which is used as a biological catalyst. There is no limitation to the type of the enzyme, any enzyme capable of reacting with aldehyde groups is included in the present invention. For example, one preferred enzyme in the present invention is transglucosidase, but the invention is not limited to it.

The complex is applied to kill bacteria or inhibit bacterial growth. There is no limitation to the type of protein, polypeptide or oligopeptide, any protein, polypeptide or oligopeptide capable of reacting with aldehyde groups is included in the present invention. For example, one preferred protein, polypeptide or oligopeptide in the present invention is polymyxin B, but the invention is not limited to it.

For example, polymyxin B can kill bacteria or inhibit bacterial growth (please refer to M. Mohorčič, I. Jerman, M. Zorko, L. Butinar, B. Orel, R. Jerala, J. Friedrich, 2010, Journal of Materials Science: Materials in Medicine, 21:2775-2782). The composite carrier described in Section [1] can be used to react with polymyxin B to form a complex for killing bacteria or inhibiting bacterial growth.

The complex is applied for affinity purification, wherein the protein, polypeptide or oligopeptide of the complex possesses a specific and reversible binding property with the corresponding bioactive substance. There is no limitation to the type of the protein, polypeptide or oligopeptide, any protein, polypeptide or oligopeptide in the prior art that can be used as a ligand in affinity chromatography and has amino groups capable of reacting with aldehyde groups is included in the present invention.

For example, glutathione, which is a corresponding ligand of glutathione S-transferase, is widely used in the affinity purification (please refer to Hong Jin, Heng Wu, Gregory Osterhaus, Jianning Wei, Kathleen Davis, Di Sha, Eric Floor, Che-Chang Hsu, Richard D. Kopke, Jang-Yen Wu, 2003, Proceedings of the National Academy of Sciences of the USA, 100:4293-4298; Cheng Xiang Hou, Zhi Qiang Fu, Byung Rae Jin, Zhong Zheng Gui, 2008, African Journal of Biotechnology, 7:311-316). The composite carrier described in Section [1] can be used to react with glutathione to form a complex for glutathione S-transferase purification.

In addition, antibody protein plays a major role in immune system. Monoclonal and polyclonal antibodies are widely used in medical diagnosis and treatment. There are many methods for preparation of monoclonal and polyclonal antibodies, all of them need to be purified beforehand. Protein A affinity chromatography column is one of the main tools for purification of the antibody (please refer to Kishore K. R. Tetala, Teris A. Van Beek, 2010, Journal of Separation Science, 33:422-438). For example, protein A is a cell wall protein of Staphylococcus aureus capable of specifically binding to the Fc region of an antibody, and its binding can be deassociated by changing the pH. Therefore, protein A affinity column is an efficient tool to purify monoclonal and polyclonal antibodies. Different from the present invention, the existing carrier of protein A affinity column is made from agarose which couples to protein A after being activated using cyanogen bromide (CNBr) under an alkaline condition (please refer to Rolf Axen, Sverker Ernback, 1971, European Journal Biochemistry, 18:351-360). Cyanogen bromide is a highly toxic compound which is dangerous in operation and transportation, and therefore limits its industrial application. However, there is no such problem with the complex formed by the composite carrier described in Section [1] and protein A.

### [6]. Methods for biocatalysis, killing bacteria and affinity chromatography

The present invention provides a biocatalytic method, wherein the complex described in Section [3] is used as a biocatalyst to catalyse the reaction of its corresponding substrates, and the protein, polypeptide or oligopeptide is enzyme.

There is no limitation to the type of the enzyme, any enzyme capable of reacting with aldehyde groups is included in the present invention. Preferably, the enzyme is transglucosidase.

The present invention provides a method for killing bacteria or inhibiting bacterial growth, wherein the complex described in Section [3] is used as a medium to kill bacteria and inhibit bacterial growth. There is no limitation to the types of protein, polypeptide or oligopeptide, any protein, polypeptide or oligopeptide capable of reacting with aldehyde groups is included in the present invention.

Preferably, the protein, polypeptide or oligopeptide of the complex in the present invention is polymyxin B.

Lastly, the present invention provides a method for affinity chromatography by using the complex described in Section [3] as a medium to purify bioactive substances. There is no limitation to the types of protein, polypeptide or oligopeptide, any protein, polypeptide or oligopeptide capable of reacting with aldehyde groups is included in the present invention.

Preferably, the protein, polypeptide or oligopeptide of the complex in the present invention is glutathione or protein A.

A specific arrangement of the preparation of the affinity column by using the composite carrier is shown as follows:
1) using porous organic foam material containing open pores as a matrix;
2) introducing reactive functional groups onto the porous organic foam material containing open pores to improve its reactivity;
3) immobilizing a protein, polypeptide or oligopeptide onto the composite carrier;
4) using the carrier containing immobilized protein, polypeptide or oligopeptide as the ligand and carrier of an affinity column.

In the present invention, melamine foam is used as the carrier, the reactivity of the melamine foam is improved by introduction of reactive functional groups so it can be used to the immobilized protein, enzyme, polypeptide or oligopeptide. The method includes following steps:
a) the porous organic foam material is cut into sheets or granules;
b) an appropriate amount of chitosan solution (0.01%-10% (w/v)) is added into the porous organic foam material, and the foam is pressed to distribute the chitosan solution evenly on the foam;
c) the carrier in step b) is immersed in an alkaline solution (0.01 M-10 M sodium hydroxide) to precipitate chitosan onto the pore walls of the porous organic foam material;
d) after a period of appropriate time, the carrier in step c) is washed with deionized water until it is in neural pH;
e) the carrier in step d) is immersed in a polyaldehyde solution (0.01%-30% (v/v));
f) after a period of appropriate time, the carrier in e) is washed with a neutral phosphate buffer (0.001 M-10 M) to remove unreacted multi-aldehyde solution.
g) a protein, enzyme, polypeptide or oligopeptide solution (0.01%-30% (w/v)) is prepared using deionized water or a buffer solution;
h) the carrier in step f) is immersed in the protein, enzyme, polypeptide or oligopeptide solution prepared in step g) for immobilization;
i) after a period of appropriate time, the carrier obtained in h) is washed with a neutral phosphate buffer (0.001 M-10 M) to remove the unreacted protein, enzyme, polypeptide or oligopeptide;
j) the immobilized protein, enzyme, polypeptide or oligopeptide is cut into disc-shaped and packed as chromatographic column, or cut into sheet-shaped and rolled into cylindrical reel structure to form a chromatographic column or immobilized enzyme column.

When adding a chitosan into the porous organic foam material, the material can be pressed by hand or by special device or machines. The crosslinking agent is generally a polyaldehyde compound (e.g. glutaraldehyde, dialdehyde starch, etc).

The examples presented below are for further illustration of the invention only and are not intended to be regarded as the limitation of invention.

### Example 1

### Immobilized transglucosidase

Twenty mg of melamine foam (Zhuhai Tin Hong Special Sponge Factory) was cut into sheet, which is used as a carrier. 0.8 g of 2% (w/v) chitosan solution (Laizhou City, Shandong Haili Biological Products Co., Ltd) was added into the foam material, followed by repeatedly pressing the carrier by hand to distribute the chitosan evenly on the carrier. The carrier was immersed in 5 ml of 1 M sodium hydroxide solution for 30 minutes and washed with deionized water until it reached neutral pH. The carrier was then immersed in 6 ml of 5% (v/v) glutaraldehyde solution (Tianjin Damao Chemical Reagent Factory) containing 1% (v/v) acetic acid for 1 hour and washed with 20 mM potassium phosphate buffer, pH 7.4, to remove the unreacted glutaraldehyde. Five hundred mg of wet carrier was obtained after removal of excess water by filter paper.

The carrier was immersed in 5 ml of 2 mg/ml transglucosidase solution (Amano Enzyme Inc., prepared in 20 mM potassium phosphate buffer, pH 7.4), for 24 hours and washed with 10 ml of 20 mM potassium phosphate buffer, pH 7.4, ten times to remove unreacted transglucosidase. Four hundred sixty mg of immobilized transglucosidase was obtained after removal of excess water by filter paper.

Ten mg of immobilized transglucosidase was weighed accurately and 10 ml of 1% (w/v) methyl-α-D-glucopyranoside solution (Sigma-Aldrich Company Ltd, prepared in 10 mM sodium acetate, pH 5.0 and pre-heated at 45°C) was added. The reaction mixture was shaken at 45°C at 180 rpm for 60 minutes and was terminated by immersing the reaction mixture in boiled water for 5 minutes.

One unit of enzyme activity was defined as 1 µg of glucose released per hour under the above condition. The amount of glucose was determined by the glucose kit of Biosino Bio-technology and Science Inc.

The specific activity of the immobilized transglucosidase in Example 1 was determined as 240,000 U/g.

### Example 2

### Immobilized polymyxin B

Forty mg of melamine foam was cut into a sheet carrier. 1.6 g of 2% (w/v) chitosan solution was added, followed by repeatedly pressing the carrier by hand to distribute the chitosan evenly on the carrier. The carrier was immersed in 10 ml of 1 M sodium hydroxide solution for 30 minutes, washed with deionized water until it reached neutral pH. The carrier was then immersed in 12 ml of 5% (v/v) glutaraldehyde solution for 1 hour and washed with 20 mM potassium phosphate buffer, pH 7.4, to remove the unreacted glutaraldehyde. One g of wet carrier was obtained after removal of excess water by filter paper.

The carrier was immersed in 20 ml of 1.8 mg/ml polymyxin B solution (World (Jiangsu) Industry Co., Ltd., prepared in 20 mM potassium phosphate buffer, pH 7.4), for 24 hours and washed with 10 ml of 20 mM potassium phosphate buffer, pH 7.4, ten times to remove the unreacted polymyxin B. One g of immobilized polymyxin B was obtained after removal of excess water by filter paper.

E. *coli* containing pREST-lac-kan (China patent CN1912127A) was inoculated into 10 ml of LB broth with 100 µg/ml kanamycin and was shaken at 37°C at 250 rpm overnight until OD₆₀₀ reached 2.5-3.0. The culture was diluted to 10⁶ cell/ml and kanamycin was added to final concentration of 100 µg/ml.

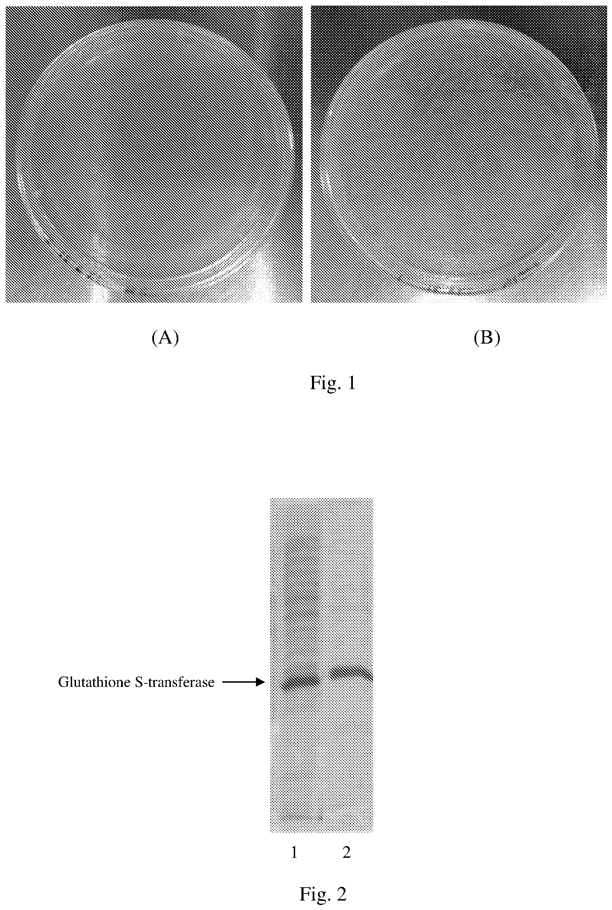

A sample containing 1 g of the immobilized polymyxin B and 10 ml of the prepared culture was named sample A. A sample containing 10 ml of the prepared culture without the immobilized polymyxin B, as the control experiment, was named sample B. Both samples A and B were incubated at 37°C at 250 rpm for 16.5 hours. The absorbance at 600 nm of samples A and B is 0 and 2.67, respectively. One hundred µl of sample A and 1µl of sample B were spread on the agar plate containing 50 µg/ml kanamycin. Results showed that there was absence of E. *coli* in sample A, which was treated with immobilized polymyxin B (Fig. 1).

### Example 3

### Immobilized glutathione

Twenty mg of melamine foam was cut into sheet carrier, with 0.8 g of 2% (w/v) chitosan solution was added, followed by repeatedly pressing the carrier by hand to distribute the chitosan evenly on the carrier. The carrier was immersed in 5 ml of 1 M sodium hydroxide solution for 30 minutes and washed with deionized water until it reached neutral pH. The carrier was then immersed in 6 ml of 5% (v/v) glutaraldehyde solution for 1 hour and washed with 20 mM potassium phosphate buffer, pH 7.4, to remove the unreacted glutaraldehyde. Five hundred mg of wet carrier was obtained after removal of excess water by filter paper.

The carrier was immersed in 30 ml of 1% (w/v) glutathione solution (Shangdong Jincheng Pharmaceutical & Chemical Co., Ltd., prepared in deionized water (pH was adjusted to 5.5)) for 24 hours and washed with 10 ml of 20 mM potassium phosphate buffer, pH 7.4, ten times to remove the unreacted glutathione. Five hundred mg of immobilized glutathione was obtained after removal of excess water by filter paper. Five hundred mg of immobilized glutathione was added to 20 ml of 2 mg/ml crude glutathione S-transferase solution (GE Healthcare, Life Sciences). The reaction mixture was incubated at 25°C at 70 rpm for 1 hour. The immobilized glutathione was washed with 10 ml of the binding buffer containing 20 mM potassium phosphate buffer, pH 7.4, five times to remove unbinded glutathione S-transferase. Five ml of the elution buffer containing 50 mM glutathione and 50mM Tris-HCl, pH 7.5 was added and incubated at 25°C at 70 rpm for 45 minutes to elute purified glutathione S-transferase. The purity of purified glutathione was greater than 90% according to the SDS-PAGE (Fig. 2).

### Example 4

### Immobilized protein A affinity column

Sixty mg of melamine foam was cut into sheet carrier, 1.2 g of 2% (w/v) chitosan solution was added, followed by repeatedly pressing the carrier by hand to distribute the chitosan evenly on the carrier. The carrier was immersed in 15 ml of 1 M sodium hydroxide solution for 30 minutes and washed with deionized water until it reached neutral pH. The carrier was then immersed in 18 ml of 5% (v/v) glutaraldehyde solution containing 1% (v/v) acetic acid for 1 hour and washed with 20 mM potassium phosphate buffer, pH 7.4, to remove the unreacted glutaraldehyde. One g of wet carrier was obtained after removal of excess water by filter paper.

The carrier was immersed in 15 ml of 5 mg/ml protein A solution (Shanghai Yaxin Biotechnology Co., Ltd., prepared in 20 mM potassium phosphate buffer, pH 7.4) for 24 hours and washed with 30 ml of 20 mM potassium phosphate buffer, pH 7.4, ten times to remove the unreacted protein A. One thousand five hundred mg of immobilized protein A was obtained after removal of the excess water by filter paper.

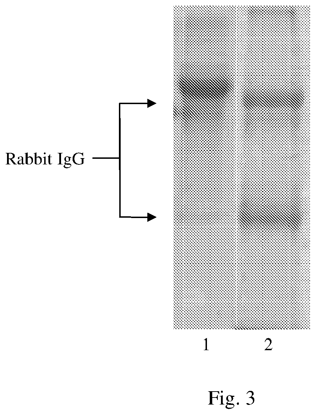

The immobilized protein A was cut into disc-shaped and packed into an affinity column of 2.6 ml. Standard rabbit serum (Shanghai Jiang Lau Biotechnology Co., Ltd.) was loaded into the protein A affinity column followed by 15 ml of the binding buffer containing 0.1 M potassium phosphate and 0.15 M sodium chloride, pH 7.2 to wash the column. Twenty ml of the elution buffer containing 0.25 M-0.1 M glycine solution and 0.02 M hydrochloric acid, pH 2.9 was used to elute purified rabbit serum (rabbit IgG). The purity of purified rabbit IgG was greater than 90% according to the SDS-PAGE (Fig. 3).

## Claims

1. A method for preparation of a porous composite carrier for immobilization of a protein, polypeptide or oligopeptide, wherein the method comprises the following steps:
a) providing a porous organic foam material containing open pores, wherein the organic foam material is melamine foam;
b) precipitating a chitosan onto a surface of walls of one or more pores of the porous organic foam material by reacting with an alkaline; and
c) crosslinking the precipitated chitosan with a polyaldehyde compound to form the composite carrier which comprises a crosslinked product having aldehyde groups and immobilized on the surface of the walls of one or more pores of the porous organic foam material, wherein the aldehyde groups are able to react with the protein, polypeptide or oligopeptide.

2. The method according to claim 1, wherein in step b), the chitosan is precipitated onto the surface by reacting with sodium hydroxide.

3. The method according to claim 1, wherein the polyaldehyde compound is glutaraldehyde or dialdehyde starch.

4. A porous composite carrier for immobilization of a protein, polypeptide or oligopeptide which is formed by the method according to any one of claims 1-3.

5. A complex, comprising:
the composite carrier according to claim 4, and
a protein, polypeptide or oligopeptide immobilized onto the composite carrier, wherein the protein, polypeptide or oligopeptide is immobilized onto the composite carrier through the reaction between the amino groups of the protein, polypeptide or oligopeptide and the aldehyde groups of the composite carrier.

6. The complex according to claim 5 wherein the protein, polypeptide or oligopeptide is an enzyme.

7. The complex according to claim 6 wherein the protein, polypeptide or oligopeptide is transglucosidase.

8. The complex according to claim 5, wherein the protein, polypeptide or oligopeptide is polymyxin B.

9. The complex according to claim 5, wherein the protein, polypeptide or oligopeptide is glutathione or protein A.

10. A method for preparing the complex according to any one of the claims 5 - 9, wherein the complex is prepared by a reaction between the composite carrier according to claim 4 and a solution of a protein, polypeptide or oligopeptide.

11. A biocatalytic method, wherein the complex according to any one of claims 5 - 7 is used as a biocatalyst to catalyse the reaction of its corresponding substrates.

12. A method for killing bacteria or inhibiting bacterial growth, wherein the complex according to claim 5, or 8, is used as a medium to kill the bacteria or inhibit bacterial growth.

13. A method for purifying a glutathione S-transferase, or monoclonal or polyclonal antibody, wherein the complex according to claim 9 is used as a medium in an affinity chromatography to purify the glutathione S-transferase, or monoclonal or polyclonal antibody.

## Patentansprüche

1. Verfahren zur Herstellung eines porösen Verbundstoffträgers zur Immobilisierung eines Proteins, Polypeptids oder Oligopeptids, wobei das Verfahren die folgenden Schritte umfasst:
a) Bereitstellen eines porösen organischen Schaumstoffmaterials, das offene Poren enthält, wobei das organische Schaumstoffmaterial Melaminschaumstoff ist;
b) Fällen eines Chitosans auf eine Oberfläche der Wände von einer oder mehreren Poren des porösen organischen Schaumstoffmaterials durch Umsetzen mit einem alkalischen Stoff; und
c) Quervernetzen des gefällten Chitosans mit einer Polyaldehydverbindung, um den Verbundstoffträger zu bilden, der ein quervernetzes Produkt mit Aldehydgruppen umfasst und auf der Oberfläche der Wände von einer oder mehreren Poren des porösen organischen Schaumstoffmaterials immobilisiert ist, wobei die Aldehydgruppen in der Lage sind, mit dem Protein, Polypeptid oder Oligopeptid zu reagieren.

2. Verfahren nach Anspruch 1, wobei in Schritt b) das Chitosan durch Umsetzen mit Natriumhydroxid auf die Oberfläche gefällt wird.

3. Verfahren nach Anspruch 1, wobei die Polyaldehydverbindung Glutaraldehyd oder Dialdehydstärke ist.

4. Poröser Verbundstoffträger zur Immobilisierung eines Proteins, Polypeptids oder Oligopeptids, der durch das Verfahren nach einem der Ansprüche 1-3 gebildet wird.

5. Komplex, umfassend:
den Verbundstoffträger nach Anspruch 4, und
ein Protein, Polypeptid oder Oligopeptid, das auf dem Verbundstoffträger immobilisiert ist, wobei das Protein, Polypeptid oder Oligopeptid durch die Reaktion zwischen den Aminogruppen des Proteins, Polypeptids oder Oligopeptids und den Aldehydgruppen des Verbundstoffträgers auf dem Verbundstoffträger immobilisiert ist.

6. Komplex nach Anspruch 5, wobei das Protein, Polypeptid oder Oligopeptid ein Enzym ist.

7. Komplex nach Anspruch 6, wobei das Protein, Polypeptid oder Oligopeptid Transglucosidase ist.

8. Komplex nach Anspruch 5, wobei das Protein, Polypeptid oder Oligopeptid Polymyxin B ist.

9. Komplex nach Anspruch 5, wobei das Protein, Polypeptid oder Oligopeptid Glutathion oder Protein A ist.

10. Verfahren zur Herstellung des Komplexes nach einem der Ansprüche 5-9, wobei der Komplex durch eine Reaktion zwischen dem Verbundstoffträger nach Anspruch 4 und einer Lösung eines Proteins, Polypeptids oder Oligopeptids hergestellt wird.

11. Biokatalytisches Verfahren, wobei der Komplex nach einem der Ansprüche 5-7 als Biokatalysator verwendet wird, um die Reaktion seiner zugehörigen Substrate zu katalysieren.

12. Verfahren zum Abtöten von Bakterien oder Hemmen von Bakterienwachstum, wobei der Komplex nach Anspruch 5 oder 8 als Mittel verwendet wird, um die Bakterien abzutöten oder Bakterienwachstum zu hemmen.

13. Verfahren zur Reinigung einer Glutathion-S-Transferase, oder eines monoklonalen oder polyklonalen Antikörpers, wobei der Komplex nach Anspruch 9 als Mittel in einer Affinitätschromatografie verwendet wird, um die Glutathion-S-Transferase, oder den monoklonalen oder polyklonalen Antikörper zu reinigen.

## Revendications

1. Procédé pour la préparation d'un support composite poreux pour l'immobilisation d'une protéine, d'un polypeptide ou d'un oligopeptide, le procédé comprenant les étapes suivantes :
a) l'utilisation d'un matériau organique poreux sous forme de mousse contenant des pores ouverts, le matériau organique sous forme de mousse étant de la mousse de mélamine ;
b) la précipitation d'un chitosane sur une surface de parois d'un ou plusieurs pores du matériau organique poreux sous forme de mousse par réaction avec une substance alcaline ; et
c) la réticulation du chitosane précipité avec un composé polyaldéhyde pour former le support composite qui comprend un produit réticulé qui a des groupes aldéhyde et qui est immobilisé sur la surface des parois d'un ou plusieurs pores du matériau organique poreux sous forme de mousse, les groupes aldéhyde pouvant réagir avec la protéine, le polypeptide ou l'oligopeptide.

2. Procédé selon la revendication 1, dans lequel dans l'étape b), le chitosane est amené à précipiter sur la surface par réaction avec de l'hydroxyde de sodium.

3. Procédé selon la revendication 1, dans lequel le composé polyaldéhyde est le glutaraldéhyde ou l'amidon dialdéhyde.

4. Support composite poreux pour l'immobilisation d'une protéine, d'un polypeptide ou d'un oligopeptide qui est formé par le procédé selon l'une quelconque des revendications 1-3.

5. Complexe, comprenant :
le support composite selon la revendication 4 et
une protéine, un polypeptide ou un oligopeptide immobilisé sur le support composite, la protéine, le polypeptide ou l'oligopeptide étant immobilisé sur le support composite par la réaction entre les groupes amino de la protéine, du polypeptide ou de l'oligopeptide et les groupes aldéhyde du support composite.

6. Complexe selon la revendication 5 dans lequel la protéine, le polypeptide ou l'oligopeptide est une enzyme.

7. Complexe selon la revendication 6 dans lequel la protéine, le polypeptide ou l'oligopeptide est la transglucosidase.

8. Complexe selon la revendication 5, dans lequel la protéine, le polypeptide ou l'oligopeptide est la polymyxine B.

9. Complexe selon la revendication 5, dans lequel la protéine, le polypeptide ou l'oligopeptide est le glutathion ou la protéine A.

10. Procédé pour la préparation du complexe selon l'une quelconque des revendications 5-9, dans lequel le complexe est préparé par une réaction entre le support composite selon la revendication 4 et une solution d'une protéine, d'un polypeptide ou d'un oligopeptide.

11. Procédé biocatalytique, dans lequel le complexe selon l'une quelconque des revendications 5-7 est utilisé en tant que biocatalyseur pour catalyser la réaction de ses substrats correspondants.

12. Procédé permettant de tuer des bactéries ou d'inhiber le développement de bactéries, dans lequel le complexe selon la revendication 5 ou 8 est utilisé en tant que milieu pour tuer les bactéries ou inhiber le développement de bactéries.

13. Procédé permettant de purifier une glutathion S-transférase ou un anticorps monoclonal ou polyclonal, dans lequel le complexe selon la revendication 9 est utilisé en tant que milieu dans une chromatographie d'affinité pour purifier la glutathion S-transférase ou l'anticorps monoclonal ou polyclonal.
